(19) Europäisches Patentamt
European Patent Office
Office européen des brevets

(11) **EP 4 148 419 A1**

(12) **EUROPEAN PATENT APPLICATION**
published in accordance with Art. 153(4) EPC

(43) Date of publication:
**15.03.2023 Bulletin 2023/11**

(51) International Patent Classification (IPC):
**G01N 21/76** (2006.01)

(21) Application number: **21799600.8**

(22) Date of filing: **08.02.2021**

(86) International application number:
**PCT/CN2021/076029**

(87) International publication number:
**WO 2021/223484 (11.11.2021 Gazette 2021/45)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.05.2020 CN 202010379814**

(71) Applicants:
• **Tangshan Food and Drug Intergration Examination and Detection Center**
  **Tangshan, Hebei 063016 (CN)**
• **Beijing Zhongjian Baotai Biotechnology Co., Ltd.**
  **Beijing 100068 (CN)**

(72) Inventors:
• **XIAO, Jin**
  **Beijing 100068 (CN)**
• **LIU, Longfei**
  **Beijing 100068 (CN)**
• **DU, Ruihuan**
  **Beijing 100068 (CN)**

(74) Representative: **Cabinet Chaillot**
  **16/20, avenue de l'Agent Sarre**
  **B.P. 74**
  **92703 Colombes Cedex (FR)**

(54) **METHOD FOR DETECTING PESTICIDE RESIDUE, AND KIT**

(57) Disclosed are a method and a kit for detecting pesticide residue, wherein an insect head preparation containing an enzyme that can be inhibited by the pesticide is used in the detection. The method has a high sensitivity with a false negative rate of 0 and a false positive rate less than 5%, and the chi-squared test showed that there was no significant difference between the detection results of the present method and those of chromatographic instrument.

Fig. 1

## Description

### Technical Field

The present invention relates to a method and a kit for detecting pesticide residue and a method for preparing the kit, and belongs to the fields of enzyme chemical analysis and biological detection.

### Background Art

Pesticide residue is a general term for pesticide protoplasts, derivatives, metabolites, degradation products and impurities that remain in the environment, organisms and food after pesticide use. Pesticide can be broadly divided into organochlorines, organophosphates, carbamates, pyrethroids, etc. according to chemical structure. Excessive pesticide residues on crops and pesticide residue metabolites in agricultural and livestock products can enter the human body through the digestive tract, causing damage to the human body to different extent and affecting people's health.

Methods for detecting pesticide residues have been developed world widely by scientists since 1950s, and common methods for detecting pesticide residues include chromatography and rapid detection methods.

Chromatography includes gas chromatography, gas chromatography-mass spectrometry, high performance liquid chromatography (HPLC), liquid chromatography-mass spectrometry, etc. Among them, the introduction of gas chromatography technology has greatly promoted the development of pesticide residue analysis and effectively improved the detection level of pesticide residue analysis; HPLC, as the fastest developing and most widely used analytical technique, can effectively separate and detect the polar pesticides with high boiling point and poor thermal stability and their metabolites, which cannot be analyzed by gas chromatography; the application of mass spectrometry as well as chromatography-mass spectrometry technology has promoted pesticide residue analysis from detection of one or several pesticides to simultaneous detection of dozens or even hundreds of different pesticides, achieving qualitative and quantitative detection of pesticides with high throughput and high sensitivity. Although chromatographic methods have accurate and reliable results with high sensitivity, they require large chromatographic and mass spectrometric instruments with high detection costs and high requirements for operators, limiting their application in agricultural product and food enterprises as well as primary laboratories.

Rapid detection methods have advantages of simple operation, short detection time, low cost and no need for large instruments, and are thus suitable for rapid screening in enterprise laboratories and primary regulatory agencies. The current rapid detection methods mainly include chemical rapid detection methods, immunoassay methods, enzymatic inhibition methods, and so on. However, the rapid detection methods have low sensitivity such that the sensitivity for many targets fails to meet the national limit requirements, and cannot be applied to a wide range of types of samples.

Therefore, there is currently a contradiction that, on the one hand, the detection of pesticide residues belongs to micro- or trace analysis, and must be achieved with high-sensitivity analytic equipment, and on the other hand, economical and convenient methods with high-sensitivity need to be adopted in enterprise laboratories and primary regulatory agencies performing large-scale detection. Generally, for experiments to improve detection sensitivity, generally considered to be adopted is further purification of reagents or samples, optimization of detection procedures, or attempt to adjustment of one or several parameters in the detection process to obtain desired improvement. However, in many practical situations, the detection of pesticide residues is not just to detect a certain individual pesticide, but may require rapid detection of two or more pesticide residues. Therefore, it is necessary to develop a rapid detection method with high sensitivity and suitable for detecting a variety of pesticides.

### Summary of the Invention

In order to address the shortcomings of the existing detection methods, the present invention provides a rapid detection method with high sensitivity and suitable for detecting a variety of pesticides and a kit for the method.

In one aspect, the present invention is based on a study on an insect head preparation. There are certain substances in insects that can be inhibited by one or more pesticides. However, the insect head has more macromolecular substances that are sensitive to pesticides, because it has many functions such as vision and touch. Therefore, the present invention chooses the insect head preparation for detection experiments.

In another aspect, the present invention is based on accidental discoveries in actual work. Over the years, the present inventor detected the residues of various pesticides in various foods based on the principles of biological enzymatic reaction and fluorescence detection, wherein many biological materials of different species have been used, such as housefly, silkworm, beetle or bee, etc. It was surprisingly found that there is a kind of bee, apis andreniformis, whose head preparation is hypersensitive to many pesticides of different kinds, such as organophosphate and carbamate pesticides and their metabolites, and the sensitivity is significantly higher than other biological materials. In particular, the head preparation is extremely sensitive to profenofos, phorate, chlorpyrifos, methiocarb, dichlorvos and/or carbaryl.

Therefore, the present invention further provides a kit and a method for sensitively and rapidly detecting organophosphate and carbamate pesticides and their metabolites.

In particular, provided are a method for detecting one or more pesticides of profenofos, phorate, chlorpyrifos, methiocarb, dichlorvos, and/or carbaryl with high-sensitivity, and a kit for the same.

The present invention further provides an optimized and stable detection kit and detection method.

The invention can be widely applied to the detection of pesticide residues in foods such as cereals, dairy products, infant foods and/or meat products.

The kit of the present invention includes a fluorescein-containing preparation, a luminescent reagent, and an insect head preparation that contains an enzyme capable of being inhibited by one or more pesticides.

Optionally, the insect is one or more of housefly, silkworm, beetle, or bee.

Preferably, the insect is apis andreniformis.

In a preferred embodiment, the fluorescein-containing preparation is D-fluorescein ethyl ester. It was found that the head preparation of apis andreniformis can react with D-fluorescein ethyl ester with high specificity. Therefore, the detection sensitivity is improved by using D-fluorescein ethyl ester instead of other preparations containing a fluorescein as the substrate.

In one embodiment, the kit of the present invention includes a head preparation of apis andreniformis, D-fluorescein ethyl ester, a luminescence reagent, a buffer A, a buffer B, a pesticide-free water, and a positive control, wherein the head preparation of apis andreniformis contains an enzyme capable of being inhibited by one or more pesticides.

In a preferred embodiment, all of the head preparation of apis andreniformis, D-fluorescein ethyl ester and the luminescent reagent are tablets. The stability and consistency of the reagents can be ensured by compressing the reagents into tablets.

Therefore, in a particularly preferred embodiment, the kit of the present invention includes tablets of the head preparation of apis andreniformis, tablets of D-fluorescein ethyl ester, tablets of luminescence reagent, a buffer A, a buffer B, a pesticide-free water, and tablets of the positive control, wherein the head preparation of apis andreniformis contains an enzyme capable of being inhibited by one or more pesticides.

In fact, the present invention provides a method for detecting pesticide residues, including using an insect head preparation containing an enzyme capbale of being inhibited by a pesticide.

The insect may be one or more of housefly, silkworm, beetle or bee; preferably, the insect is apis andreniformis.

The pesticide is one or more of organophosphate and/or carbamate pesticides; more preferably, the pesticide is one or more of profenofos, phorate, chlorpyrifos, methiocarb, dichlorvos, and/or carbaryl.

More specifically, the present invention provides a method for detecting pesticide residues, including using a fluorescein-containing preparation, a luminescent reagent, and an insect head preparation containing an enzyme capable of being inhibited by one or more pesticides.

Preferably, all of the head preparation of apis andreniformis, D-fluorescein ethyl ester and the luminescent reagent are tablets.

In a particularly preferred embodiment, the method of the present invention includes using tablets of the head preparation of apis andreniformis, tablets of D-fluorescein ethyl ester, tablets of the luminescent reagent, a buffer A, a buffer B, a pesticide-free water and tablets of the positive control, wherein the head preparation of apis andreniformis contains an enzyme capable of being inhibited by one or more pesticides.

It can be seen that the method of the present invention includes using the kit of the present invention.

**Description of the Drawings**

Fig. 1 showed 40% inhibition of different kinds of bees to pesticides. The upper curve is the result of apis mellifera ligustica, the middle curve is the result of apis cerana cerana, and the lower curve is the result of apis andreniformis.

Fig. 2 showed the relationship between D-fluorescein ethyl ester and RLU.

Fig. 3 showed the relationship between incubation temperature and RLU.

Fig. 4 showed the relationship between reaction time and RLU.

Fig. 5 showed the relationship between incubation time and RLU.

**Detailed Description**

Hereinafter, the method and kit of the present invention will be illustrated in detail by the following examples. It will be easily understood by those skilled in the art that the scope covered by this application is not limited to the specific examples of the method, kit, and step described in the specification, and equivalent substitutions and known modifications thereon are intended to be encompassed by the disclosure of the present invention.

**Example 1 Reagent preparation**

1. Preparation of head preparation of apis andreniformis:

1) Apis andreniformis heads were collected and placed into a 50 mL beaker containing approximately 15 mL of an ice-cold 0.07 M phosphate buffer (pH 7.0, containing 1 mM EDTA and 1 μM phenylthiourea). The apis andreniformis heads were slowly homogenized in a homogenizer at 100 rpm.

2) The homogenate was transferred to a centrifuge tube and centrifuged at 3000 ± 100 rpm, and the supernatant was collected.

3) The supernatant was added to a Sephadex G-25 column equilibrated with a 0.07M phosphate buffer (pH 7.0, containing 1 mM EDTA and 1 M phenylthiourea). Fractions were collected every 2 minutes, and their esterase activity was detected by using fluorescein acetate as the substrate, ATP and luciferase as the luminescent agent, and their bioluminescence was determined. The fractions having esterase activity were combined, and optionally, further prepared into compressed tablets containing inert filler as the head preparation of apis andreniformis for pesticide residue detection, and the protein content in each tablet was 4-20 mg.

2. Preparation of D-fluorescein ethyl ester:
74ng of D-fluorescein ethyl ester was weighed and optionally, prepared into compressed tablets containing inert filler, wherein the content of D-fluorescein ethyl ester in each tablet was 0.24 nmol.

3. Preparation of the luminescent reagent:

1) 328.65 ng of ATP, 13.146 μg of magnesium sulfate and 10μg of luciferase were weighed and mixed well.
2) Optionally, the mixture was prepared into compressed tablets containing inert filler as the luminescent reagent for pesticide residue detection.

4. Preparation of the buffer A:
A 0.07M phosphate buffer at pH 7.0 was prepared, and added with EDTA and phenylthiourea with their concentrations of 1mM and 1μM, respectively.

5. Preparation of the buffer B:
4.48g of trimethyl amino acid, 0.6g of magnesium sulfate, 0.146g of EDTA, 100mg of bovine serum albumin and 77mg of dithiothreitol were weighed and added into 600 mL of water, adjusted pH to 7.8 with 10% sodium hydroxide, and diluted to 1L with distilled water.

**Example 2 Sample preparation**

1. Sterilized milk sample
100μL of milk was pipetted into 1.0mL of pesticide-free water and mixed well.
2. Raw milk sample
1mL of raw milk was pipetted to a centrifuge tube, heated at 85±2 °C for 5 minutes, and quickly cooled to 0-4 °C. 100μL of the milk was pipetted into 1.0mL of pesticide-free water and mixed well.
3. Cereal/meat product samples

a) For cereals, 100g of a cereal sample was fully pulverized or ground; and for meat products, 50g of a lean tissue was taken and added to a food mixer/grinder, added with 100 mL of a sodium sulfate solution and stirred at a high speed for 1 minute.

b) 0.5 g of the sample in step a) was mixed with 2 mL of the sodium sulfate solution, and added with 2 mL of an ethyl acetate-acetone solution. 2mL of the sodium sulfate and 2mL of the ethyl acetate-acetone solution were used as the blank control. The mixture was centrifuged at 1200±200g for 2 minutes, and 50μL of the upper organic phase was pipetted, dried with nitrogen at 45±1°C, re-dissolved with 1mL of pesticide-free water, and mixed well on a shaker.

4. Infant food samples

1g of an infant food was mixed with 1mL of pesticide-free water, added with 2mL of the ethyl acetate-acetone solution, and centrifuged at 1200±200g for 3 minutes. 2mL of the pesticide-free water and 2mL of the ethyl acetate-acetone solution were used as blank control. 50 μL of the upper organic phase was pipetted, dried with nitrogen, re-dissolved with 0.5mL of the pesticide-free water, and mixed well on a shaker.

**Example 3 Detection process**

1. Detection procedures

1) Preparation of a solution of the apis andreniformis head preparation: 1 mL of the buffer A and a apis andreniformis head preparation tablet were added into a clean microtube, mixed well, and stood for 30 seconds to allow the solid matter to settle to the bottom of the microtube.

2) 50 μL of the prepared solution of the apis andreniformis head preparation was added to 100 μL of the sample or the blank control, mixed 3 times on a shaker, and incubated at 35±1°C for 10 minutes.

3) When the incubation was performed for 8 minutes, a solution of D-fluorescein ethyl ester was prepared. 1 mL of pesticide-free water and a D-fluorescein-ethyl ester tablet were added into a clean microtube, mixed well, and stood for 30 seconds to allow the solid matter to settle to the bottom of the microtube.

4) After the 10-minute incubation was completed, 50μL of the prepared D-fluorescein ethyl ester solution was directly added to the bottom of each microtube in the incubator, mixed well and incubated at 35±1°C for 5 minutes.

5) The luminescence reagent was prepared immediately after the incubation started: 10 mL of the buffer B and a luminescence reagent tablet was mixed well and stood to allow the solid matter to settle.

6) At the end of the 5-minute incubation, the microtube was taken from the incubator and added with 1 mL of the luminescent reagent, and the microtube was capped.

7) The data were read on a rapid biofluorescence detector, and the RLU value of each microtube was recorded in turn.

2. Result calculation

The inhibition (%) was calculated following the equation below:

$$\text{Inhibition} (\%) = [(A0-A1)/A0]*100$$

wherein, A0 is the RLU value of blank control, A1 is the RLU value of the sample to be tested.

3. Result judgment

When the inhibition of the sample is less than 40%, it indicates that the pesticide in the sample is negative.
When the inhibition of the sample is 40% or more, it indicates that the pesticide in the sample is positive. In this case, other methods (such as chromatography) can be used according to actual needs for further re-detection verification or confirmation of the specific type and precise content of the pesticide.

**Example 4 Ultra-high sensitivity of apis andreniformis to a variety of pesticides**

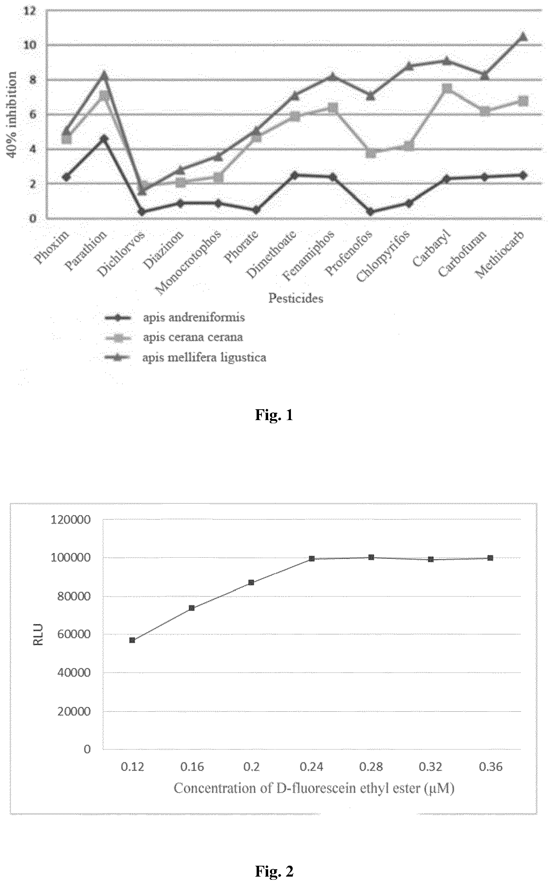

In order to intuitively reflect the specific sensitivity of apis andreniformis to pesticides, this example directly selected apis cerana cerana and apis mellifera ligustica that are closest to apis andreniformis in their biological classifications, instead of other insect species, to perform parallel experiments. Bee head preparations were prepared following the method in "Preparation of head preparation of apis andreniformis" of Example 1, standard sample of pesticide with pesticide-free water were detected following the above detection procedures and blank control was also tested, and the esterase activity in the bee head preparations was calculated under 40% inhibition. The detection results are shown in Table 1 and Fig. 1 (Fig. 1 was originally a color image, which was gray-scale processed).

It can be seen that the concentration of different kinds of organophosphate and carbamate pesticides under 40% inhibition on apis andreniformis is significantly lower than those on apis cerana cerana and apis mellifera ligustica, that is, apis andreniformis is very sensitive to various pesticides. In particular, apis andreniformis showed extremely significant sensitivity to profenofos, phorate, chlorpyrifos, methiocarb, dichlorvos and/or carbaryl with 40% inhibition at a concentration of 0.4, 0.5, 0.9, 2.5, 0.4, 2.3 μg/kg respectively. Compared with the corresponding concentrations of these pesticides at 40% inhibition on apis cerana ceranas and apis mellifera ligusticas, apis andreniformiss has sensitivity to these pesticides about 3-5 times higher than those on apis cerana ceranas and apis mellifera ligusticas.

Table 1 Types and concentrations of pesticides under 40% inhibition

| Pesticides | apis andreniformis (μg/kg) | apis cerana cerana (μg/kg) | apis mellifera ligustica (μg/kg) |
|---|---|---|---|
| Phoxim | 2.4 | 4.6 | 5.1 |
| Parathion | 4.6 | 7.1 | 8.3 |
| Dichlorvos | 0.4 | 1.9 | 1.6 |
| Diazinon | 0.9 | 2.1 | 2.8 |
| Monocrotophos | 0.9 | 2.4 | 3.6 |
| Phorate | 0.5 | 4.7 | 5.1 |
| Dimethoate | 2.5 | 5.9 | 7.1 |
| Fenamiphos | 2.4 | 6.4 | 8.2 |
| Profenofos | 0.4 | 3.8 | 7.1 |
| Chlorpyrifos | 0.9 | 4.2 | 8.8 |
| Carbaryl | 2.3 | 7.5 | 9.1 |
| Carbofuran | 2.4 | 6.2 | 8.3 |
| Methiocarb | 2.5 | 6.8 | 10.5 |

**Example 5 Optimal concentration of D-fluorescein ethyl ester**

100 μL of blank control was mixed well with 50 μL of the apis andreniformis head preparation solution, added with 50μL of D-fluorescein ethyl ester at concentrations of 0.12, 0.16, 0.20, 0.24, 0.28, 0.32 and 0.36 μM respectively, incubated for 5 minutes, added with the luminescent reagent solution, and the RLU values were read immediately on a photometer. Each concentration was measured 6 times, and the average value was taken. The detection results are shown in Fig. 2.

It can be seen that the highest RLU value was obtained when the concentration of the added D-fluorescein ethyl ester was 0.24 μM, indicating that 0.24 μM is the highest concentration that can react with 50 μL of the apis andreniformis head preparation. Since D-fluorescein ethyl ester was diluted by 1 mL of the pesticide-free water and 50 μL was taken for each reaction, the content of D-fluorescein ethyl ester in the tablet was 0.24 nmol.

**Example 6 Optimal incubation temperature**

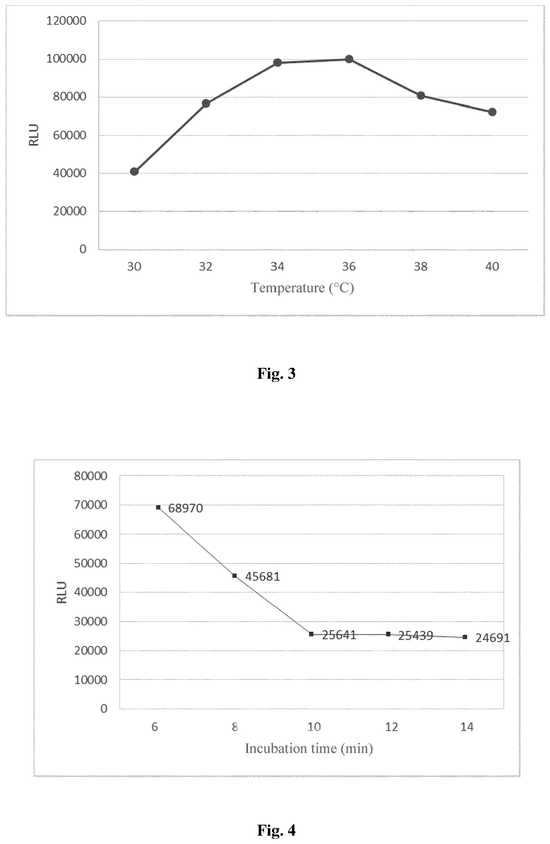

100 μL of the blank control was mixed well with 50 μL of the apis andreniformis head preparation solution, added with 50 μL of D-fluorescein ethyl ester, incubated at 30, 32, 34, 36, 38 and 40 °C respectively for 5 minutes, added with the luminescent reagent solution, and the RLU values were read immediately on a photometer. Each temperature was measured 6 times, and the average value was taken. The detection results are shown in Fig. 3.

It can be seen that RLU increases first and then decreases as the temperature increases, and when the temperature is 34-36 °C, RLU reaches the highest point and then gradually decreases. Therefore, the Apis andreniformis head preparation has the highest activity at 35±1 °C, which is the optimal incubation temperature.

**Example 7 Optimal reaction time between the apis andreniformis head preparation solution and the sample mixture**

Phorate standard was added to the milk sample to obtain a milk sample with a concentration of 10 μg/L. 100 μL of the sample was mixed with 50 μL of the apis andreniformis head preparation solution, incubated for 6, 8, 10, 12 and 14 minutes respectively, added with D-fluorescein ethyl ester and the luminescent reagent solution following the above detection procedures, and the RLU values were finally measured on a photometer. Each reaction time was measured 6 times, and the average value was taken. The detection results are shown in Fig. 4.

It can be seen that when the temperature was 35°C, RLU gradually decreased as the incubation time extended, and after 10 minutes of incubation, RLU did not decrease significantly, indicating that the reaction has been completed after 10 minutes of incubation. Therefore, the optimal reaction time between the apis andreniformis head preparation solution and the sample mixture is 10 minutes.

**Example 8 Optimal incubation time of the apis andreniformis head preparation solution and D-fluorescein ethyl ester**

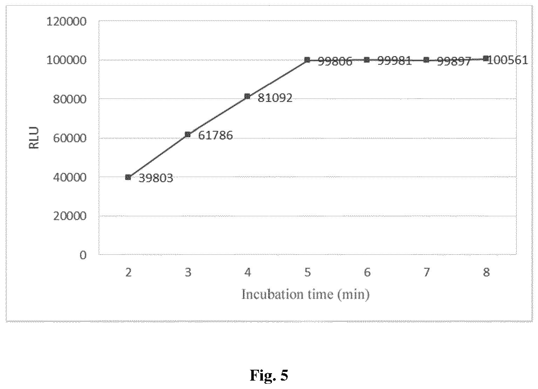

100 μL of the blank control was mixed well with 50 μL of the apis andreniformis head preparation solution, added with 50 μL of D-fluorescein ethyl ester, incubated at 35 °C for 2, 3, 4, 5, 6, 7 and 8 minutes respectively, added with the luminescent reagent solution, and the RLU values were read immediately on a photometer. Each incubation time was measured 6 times, and the average value was taken. The test results are shown in Fig. 5. It can be seen that the optimal incubation time is 5 minutes.

**Example 9 False negative rate, false positive rate and specificity of the method of the present invention**

200 samples of cereals, dairy products, meat products and infant formula foods were detected by the method of the present invention. According to the "Technical Specification for Evaluation of Food Fast Testing Methods", the detection results are shown in Tables 2-5.

Table 2 Cereals

| Samples | Detection results | | Total |
|---|---|---|---|
| | Positive | Negative | |
| Positive | 100 | 0 | 100 |
| Negative | 3 | 97 | 100 |
| Total | 103 | 97 | 200 |
| Specificity | p-=97/100=97% | | |
| False negative rate | pf-=0/100=0 | | |
| False positive rate | pf+=3/100=3% | | |

Table 3 Dairy products

| Samples | Detection results | | Total |
|---|---|---|---|
| | Positive | Negative | |
| Positive | 100 | 0 | 100 |
| Negative | 2 | 98 | 100 |
| Total | 102 | 98 | 200 |
| Specificity | p-=98/100=98% | | |
| False negative rate | pf-=0/100=0% | | |
| False positive rate | pf+=2/100=2% | | |

Table 4 Meat products

| Samples | Detection results | | Total |
|---|---|---|---|
| | Positive | Negative | |
| Positive | 100 | 0 | 100 |
| Negative | 2 | 98 | 100 |
| Total | 102 | 98 | 200 |
| Specificity | p-=98/100=98% | | |
| False negative rate | pf-=0/100=0% | | |
| False positive rate | pf+=2/100=2% | | |

Table 5 Infant formula food

| Samples | Detection results | | Total |
|---|---|---|---|
| | Positive | Negative | |
| Positive | 100 | 0 | 100 |
| Negative | 3 | 97 | 100 |
| Total | 103 | 97 | 200 |
| Specificity | p-=97/100=97% | | |
| False negative rate | pf-=0/100=0% | | |
| False positive rate | pf+=3/100=3% | | |

It can be seen from Tables 2-5 that for various samples, the false negative rate was 0, the false positive rate was less than 5%, and the specificity was greater than 95%.

**Example 10 Consistency of the detection results of the kit of the present invention and the chromatographic instrument**

The kit of the present invention and a chromatographic instrument were used to detect 50 samples of wheat, milk, pork and infant formula milk powder respectively (200 samples in total), and the results were compared.
When the detection result is lower than the detection limit, it is represented by "-" for the method of the present invention, and by "ND" for the chromatographic instrument; and when the detection result is higher than the detection limit, that is, when the result of the method of the present invention is positive, it is represented by "+", while the instrument method displays the specific value of the content. The comparison results are shown in Tables 6-9.

Table 6 Comparison results of the method of the present invention and the instrument method (wheat) ($\mu$g/kg)

| Sample | 1 | 2 | 3 | 4 | 5 | 6 | 7 | 8 | 9 | 10 |
|---|---|---|---|---|---|---|---|---|---|---|
| Rapid method | - | - | + | - | - | - | - | - | - | - |
| Instrument method | ND | ND | Parathion 105.1 | ND | ND | ND | ND | ND | ND | ND |
| Sample | 11 | 12 | 13 | 14 | 15 | 16 | 17 | 18 | 19 | 20 |
| Rapid method | + | - | - | - | - | - | - | - | - | - |
| Instrument method | Carbaryl 29.8 | ND | ND | ND | ND | ND | ND | ND | ND | ND |
| Sample | 21 | 22 | 23 | 24 | 25 | 26 | 27 | 28 | 29 | 30 |
| Rapid method | - | - | - | + | - | - | - | - | - | + |
| Instrument method | ND | ND | ND | Moncrotophos 26.9 | ND | ND | ND | ND | ND | ND |
| Sample | 31 | 32 | 33 | 34 | 35 | 36 | 37 | 38 | 39 | 40 |
| Rapid method | - | - | - | - | - | - | - | - | - | - |
| Instrument method | ND | ND | ND | ND | ND | ND | ND | ND | ND | ND |
| Sample | 41 | 42 | 43 | 44 | 45 | 46 | 47 | 48 | 49 | 50 |
| Rapid method | - | + | - | - | - | + | - | - | - | - |
| Instrument method | ND | Parathion 129.1 | ND | ND | ND | Carbofuran 62.5 | ND | ND | ND | ND |

Table 7 Comparison results of the method of the present invention and the instrument method (milk) ($\mu$g/kg)

| Sample | 1 | 2 | 3 | 4 | 5 | 6 | 7 | 8 | 9 | 10 |
|---|---|---|---|---|---|---|---|---|---|---|
| Rapid method | - | - | + | - | - | - | - | - | + | - |
| Instrument method | ND | ND | Dichlorvos 112.3 | ND | ND | ND | ND | ND | Diazinon 129.8 | ND |
| Sample | 11 | 12 | 13 | 14 | 15 | 16 | 17 | 18 | 19 | 20 |
| Rapid method | - | - | - | - | - | - | - | - | - | - |
| Instrument method | ND | ND | ND | ND | ND | ND | ND | ND | ND | ND |
| Sample | 21 | 22 | 23 | 24 | 25 | 26 | 27 | 28 | 29 | 30 |
| Rapid method | - | - | - | + | - | - | - | - | - | + |
| Instrument method | ND | ND | ND | Phorate 125.1 | ND | ND | ND | ND | ND | ND |
| Sample | 31 | 32 | 33 | 34 | 35 | 36 | 37 | 38 | 39 | 40 |
| Rapid method | + | - | - | - | - | - | - | - | - | - |
| Instrument method | Chorpyrifos 31.7 | ND | ND | ND | ND | ND | ND | ND | ND | ND |
| Sample | 41 | 42 | 43 | 44 | 45 | 46 | 47 | 48 | 49 | 50 |
| Rapid method | - | - | + | - | - | - | - | - | - | - |
| Instrument method | ND | ND | Chorpyrifos 21.5 | ND | ND | ND | ND | ND | ND | ND |

Table 8 Comparison results between the method of the present invention and the instrument method (pork) ($\mu$g/kg)

| Sample | 1 | 2 | 3 | 4 | 5 | 6 | 7 | 8 | 9 | 10 |
|---|---|---|---|---|---|---|---|---|---|---|
| Rapid method | - | - | - | + | - | - | - | - | - | + |
| Instrument method | ND | ND | ND | Dimethoate 51.8 | ND | ND | ND | ND | ND | Carbofuran 58.9 |
| Sample | 11 | 12 | 13 | 14 | 15 | 16 | 17 | 18 | 19 | 20 |
| Rapid method | - | - | - | - | - | + | - | - | - | - |
| Instrument method | ND | ND | ND | ND | ND | ND | ND | ND | ND | ND |
| Sample | 21 | 22 | 23 | 24 | 25 | 26 | 27 | 28 | 29 | 30 |
| Rapid method | - | - | - | + | - | - | - | - | - | - |
| Instrument method | ND | ND | ND | Carbofuran 12.9 | ND | ND | ND | ND | ND | ND |
| Sample | 31 | 32 | 33 | 34 | 35 | 36 | 37 | 38 | 39 | 40 |

(continued)

| Sample | 1 | 2 | 3 | 4 | 5 | 6 | 7 | 8 | 9 | 10 |
|---|---|---|---|---|---|---|---|---|---|---|
| Rapid method | - | - | - | - | + | - | - | - | - | - |
| Instrument method | ND | ND | ND | ND | Carbaryl 61.7 | ND | ND | ND | ND | ND |
| Sample | 41 | 42 | 43 | 44 | 45 | 46 | 47 | 48 | 49 | 50 |
| Rapid method | - | - | - | - | - | + | - | - | - | - |
| Instrument method | ND | ND | ND | ND | ND | Dichlorvos 12.7 | ND | ND | ND | ND |

Table 9 Comparison results of the method of the present invention and the instrument method (infant formula food) (μg/kg)

| Sample | 1 | 2 | 3 | 4 | 5 | 6 | 7 | 8 | 9 | 10 |
|---|---|---|---|---|---|---|---|---|---|---|
| Rapid method | - | - | - | - | - | - | + | - | - | + |
| Instrument method | ND | ND | ND | ND | ND | ND | Diazinon 25.3 | ND | ND | Carbaryl 56.72 |
| Sample | 11 | 12 | 13 | 14 | 15 | 16 | 17 | 18 | 19 | 20 |
| Rapid method | - | - | - | - | - | + | - | - | - | - |
| Instrument method | ND | ND | ND | ND | ND | Dichlorvos 60.64 | ND | ND | ND | ND |
| Sample | 21 | 22 | 23 | 24 | 25 | 26 | 27 | 28 | 29 | 30 |
| Rapid method | - | - | - | - | - | - | - | - | - | - |
| Instrument method | ND | ND | ND | ND | ND | ND | ND | ND | ND | ND |
| Sample | 31 | 32 | 33 | 34 | 35 | 36 | 37 | 38 | 39 | 40 |
| Rapid method | - | - | + | - | - | - | - | - | - | - |
| Instrument method | ND | ND | Diazinon 17.8 | ND | ND | ND | ND | ND | ND | ND |
| Sample | 41 | 42 | 43 | 44 | 45 | 46 | 47 | 48 | 49 | 50 |
| Rapid method | - | - | - | - | - | - | - | - | - | - |
| Instrument method | ND | ND | ND | ND | ND | ND | ND | ND | ND | ND |

It can be seen from Tables 6-9 that the chromatographic instrument detected 19 positive samples, which were also all positive by detected by the kit of the present invention; the chromatographic instrument detected 181 negative samples, which showed that 178 samples were negative and 3 samples were positive after detected by the kit of the present invention. That is, the method of the present invention has high sensitivity and no false negative.

Table 10 The experimental data distribution table of the method of the present invention and the instrument method

| Detection results of chromatographic instrument | Detection results of the kit of the present invention | | Total |
|---|---|---|---|
| | Positive | Negative | |
| Positive | 19 | 0 | 19 |

(continued)

| Detection results of chromatographic instrument | Detection results of the kit of the present invention | | Total |
| --- | --- | --- | --- |
| | Positive | Negative | |
| Negative | 3 | 178 | 181 |
| Total | 22 | 178 | 200 |

The chi-square test was performed on the detection results of the above two methods and the significant difference ($\chi$2) can be calculated from Table 10 as:

$$\chi^2 = (|0\text{-}3|\text{-}1)^2 / (0+3) = 1.33$$

$\chi^2 < 3.84$, indicating that there is no significant difference between the positive confirmation ratio detected by the kit of the present invention and the chromatographic instrument within the 95% confidence interval.

In summary, apis andreniformis head preparation used in the present invention can react with a broad-spectrum of pesticides with a strong binding ability, and a broad-spectrum of pesticides and their metabolites can be accurately detected with very high sensitivity. Preferably, the substrate adopts D-fluorescein ethyl ester that is very sensitive to the apis andreniformis head preparation, so that the detection sensitivity is higher; and the reagents are prepared into compressed tablets to ensure the stability and consistency of the reagents.

Although the invention and its advantages have been described in detail, it should be understood that various modifications, substitutions and transformations can be made without going beyond the spirit and scope of the invention defined by the appended claims.

**Claims**

1. A kit comprising a fluorescein-containing preparation, a luminescent reagent and an insect head preparation, wherein the insect head preparation contains a substance capable of being inhibited by a pesticide.

2. The kit according to claim 1, wherein the insect is bee; preferably, the bee is apis andreniformis.

3. The kit according to claim 2, wherein the pesticide is one or more of organophosphate and/or carbamate pesticides.

4. The kit according to claim 3, wherein the pesticide is one or more of profenofos, phorate, chlorpyrifos, methiocarb, dichlorvos and/or carbaryl.

5. The kit according to any one of claims 1-4, wherein the fluorescein-containing preparation is D-fluorescein ethyl ester.

6. The kit according to claim 6, wherein all of the fluorescein-containing preparation, the luminescent reagent and the insect head preparation are tablets.

7. The kit according to any one of claims 1-6 for use in detection of pesticide residue.

8. A method for detecting pesticide residue comprising using an insect head preparation containing an enzyme capable of being inhibited by the pesticide in the detection.

9. The method according to claim 8, wherein the insect is bee; preferably, the insect is apis andreniformis.

10. The method according to claim 8 or 9, wherein the pesticide is an organophosphate and/or carbamate pesticide; more preferably, the pesticide is one or more of profenofos, phorate, chlorpyrifos, methiocarb, dichlorvos and/or carbaryl.

Fig. 1

Fig. 2

**Fig. 3**

**Fig. 4**

**Fig. 5**

## INTERNATIONAL SEARCH REPORT

| | International application No. |
| --- | --- |
| | **PCT/CN2021/076029** |

| | |
| --- | --- |
| **A.** | **CLASSIFICATION OF SUBJECT MATTER** |
| | G01N 21/76(2006.01)i |
| | |
| | According to International Patent Classification (IPC) or to both national classification and IPC |

| | |
| --- | --- |
| **B.** | **FIELDS SEARCHED** |

Minimum documentation searched (classification system followed by classification symbols)

    G01N 21, C12Q 1, G01N 31

Documentation searched other than minimum documentation to the extent that such documents are included in the fields searched

Electronic data base consulted during the international search (name of data base and, where practicable, search terms used)

    CNKI, CNTXT, VEN, 农药, 荧光素, 荧光素酶, ATP, 蜜蜂, 乙酰胆碱酯酶, pesticide, luciferin, luciferase, bee, acetylcholinesterase, Ache

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| PX | CN 111272726 A (BEIJING ZHONGJIAN BAOTAI BIOTECHNOLOGY CO., LTD.) 12 June 2020 (2020-06-12)<br>    claims 1-9 | 1-10 |
| X | US 5374535 A (CHARM SCI INC.) 20 December 1994 (1994-12-20)<br>    description, column 5 line 40 - column 12 line 26 | 1-10 |
| Y | CN 103389301 A (LI, Huanfa et al.) 13 November 2013 (2013-11-13)<br>    description, paragraphs 5-10 | 1-7 |
| Y | CN 1734257 A (SHANGHAI INSTITUTES FOR BIOLOGICAL SCIENCES, CHINESE ACADEMY OF SCIENCES) 15 February 2006 (2006-02-15)<br>    description page 3 paragraphs 2-12 | 1-7 |
| X | CN 1734257 A (SHANGHAI INSTITUTES FOR BIOLOGICAL SCIENCES, CHINESE ACADEMY OF SCIENCES) 15 February 2006 (2006-02-15)<br>    description page 3 paragraphs 2-12 | 8-10 |

☑ Further documents are listed in the continuation of Box C.      ☑ See patent family annex.

| | |
| --- | --- |
| \*   Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
| --- | --- |
| **12 April 2021** | **23 April 2021** |

| Name and mailing address of the ISA/CN | Authorized officer |
| --- | --- |
| **China National Intellectual Property Administration (ISA/CN)**<br>**No. 6, Xitucheng Road, Jimenqiao, Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

| International application No. |
|---|
| **PCT/CN2021/076029** |

### C.    DOCUMENTS CONSIDERED TO BE RELEVANT

| Category* | Citation of document, with indication, where appropriate, of the relevant passages | Relevant to claim No. |
|---|---|---|
| Y | 姚冰 等 (YAO, Bing et al.). "基于荧光素酶发光体系测试饮水中农药的综合毒性 ( Determination of Synthetic Toxicity of Pesticides in Drinking Water Based on Inhibition of Bioluminescence from Luciferase Reacting System)" *环境监测管理与技术 (The Administration and Technique of Environmental Monitoring),* Vol. 22, No. 1, 28 February 2010 (2010-02-28), pp. 37-40 | 1-7 |
| Y | 张莹 等 (ZHANG, Ying et al.). "两种蜜蜂头部乙酰胆碱酯酶对杀虫药剂敏感度比较 ( Comparison of the Head Acetylcholinesterase Sensitivity to Insecticides between Two Honeybee Populations)" *农药学学报 (Chinese Journal of Pesticide Science),* Vol. 7, No. 3, 30 September 2005 (2005-09-30), pp. 221-226 | 1-7 |
| X | 张莹 等 (ZHANG, Ying et al.). "两种蜜蜂头部乙酰胆碱酯酶对杀虫药剂敏感度比较 ( Comparison of the Head Acetylcholinesterase Sensitivity to Insecticides between Two Honeybee Populations)" *农药学学报 (Chinese Journal of Pesticide Science),* Vol. 7, No. 3, 30 September 2005 (2005-09-30), pp. 221-226 | 8-10 |
| A | CN 102636482 A (CHINA AGRICULTURAL UNIVERSITY) 15 August 2012 (2012-08-15) entire document | 1-10 |

Form PCT/ISA/210 (second sheet) (January 2015)

**INTERNATIONAL SEARCH REPORT**
Information on patent family members

| International application No. |
| --- |
| **PCT/CN2021/076029** |

| Patent document cited in search report | | | Publication date (day/month/year) | Patent family member(s) | | | Publication date (day/month/year) |
| --- | --- | --- | --- | --- | --- | --- | --- |
| CN | 111272726 | A | 12 June 2020 | CN | 111272726 | B | 09 March 2021 |
| US | 5374535 | A | 20 December 1994 | JPH | 0650588 | A | 07 July 1994 |
| | | | | EP | 0576667 | A1 | 05 January 1994 |
| | | | | US | 5283180 | A | 01 February 1994 |
| | | | | EP | 0576667 | B1 | 04 April 2001 |
| | | | | CA | 2105770 | C | 22 September 1998 |
| | | | | AU | 667518 | B2 | 28 March 1996 |
| | | | | AU | 3584693 | A | 03 August 1993 |
| | | | | US | 5374534 | A | 20 December 1994 |
| | | | | DE | 69330077 | D1 | 10 May 2001 |
| | | | | WO | 9314222 | A1 | 22 July 1993 |
| CN | 103389301 | A | 13 November 2013 | | None | | |
| CN | 1734257 | A | 15 February 2006 | CN | 100520373 | C | 29 July 2009 |
| CN | 102636482 | A | 15 August 2012 | | None | | |

Form PCT/ISA/210 (patent family annex) (January 2015)